# EUROPEAN PATENT SPECIFICATION

(11) **EP 1 072 688 B1**
(45) Date of publication and mention of the grant of the patent: **30.06.2004**
(21) Application number: 98914021.5
(22) Date of filing: 15.04.1998
(51) Int. Cl.: C12N 15/10

(54) **METHOD FOR IN VIVO DETECTING A TARGET PROTEIN GENE BY A DRUG**
VERFAHREN ZUR BESTIMMUNG DES GENS EINES ZIELPROTEINS MIT HILFE EINES MEDIKAMENTES
PROCEDE DE DETECTION IN VIVO D'UN GENE PROTEIQUE CIBLE A L'AIDE DE MEDICAMENT

(43) Date of publication of application: 31.01.2001
(73) Proprietor: Hidaka, Hiroyoshi, Nagoya-shi, Aichi 468-0063 (JP)
(72) Inventor: HIDAKA, Hiroyoshi, Nagoya-shi, Aichi 468-0063 (JP); TANAKA, Hideki, Nagoya-shi, Aichi 460-0012 (JP)
(74) Representative: HOFFMANN - EITLE
(86) International application number: PCT/JP1998/001712
(87) International publication number: WO 1999/053094

(56) References cited:
- EP-A- 0 754 682
- WO-A-96/31625
- WO-A-99/45143
- SPARKS A B ET AL: "CLONING OF LIGAND TARGETS: SYSTEMATIC ISOLATION OF SH3 DOMAIN- CONTAINING PROTEINS" NATURE BIOTECHNOLOGY, NATURE PUBLISHING, US, vol. 14, no. 6, June 1996 (1996-06), pages 741-744, XP000876847 ISSN: 1087-0156
- TANAKA HIDEKI ET AL: "Isolation of cDNAs encoding cellular drug-binding proteins using a novel expression cloning procedure: Drug-western." MOLECULAR PHARMACOLOGY, vol. 55, no. 2, February 1999 (1999-02), pages 356-363, XP002192762 ISSN: 0026-895X
- CWIRLA S E ET AL: "PEPTIDES ON PHAGE: A VAST LIBRARY OF PEPTIDES FOR IDENTIFYING LIGANDS" PROCEEDINGS OF THE NATIONAL ACADEMY OF SCIENCES OF USA, NATIONAL ACADEMY OF SCIENCE. WASHINGTON, US, vol. 87, no. 16, 1 August 1990 (1990-08-01), pages 6378-6382, XP000141872 ISSN: 0027-8424
- BOECKLER CHRISTOPHE ET AL: "Immunogenicity of new heterobifunctional cross-linking reagents used in the conjugation of synthetic peptides to liposomes." JOURNAL OF IMMUNOLOGICAL METHODS, vol. 191, no. 1, 1996, pages 1-10, XP004020848 ISSN: 0022-1759
- THE PHARMACEUTICAL MONTHLY, Vol. 36(10), (1994), HIROYOSHI HIDAKA, "Special Issue on 'The 5th Symposium on Clinical Pharmacy' the Forefront of Molecular Pharmacology (in Japanese)", pages 2245-2250, XP002921504.
- AGRIC. BIOL. CHEM., Vol. 51(2), (1987), N. IWATSUKI et al., "A Complete Rat Serum Albumin cDNA Clone Directly Identified by Immunological Screening of cDNA Expression Library", pages 379-384, XP002921505.
- MOL. GEN. GENET., Vol. 213, (1988), G. HOULNE et al., "Characterization of cDNA Sequences for LHCI Apoproteins in Euglena Gracilis: The mRNA Encodes a Large Precursor Containing Several Consecutive Divergent Polypeptides", pages 479-486, XP002921506.
- ANAL. BIOCHEM., Vol. 209(2), (1993), T.S. McCLINTOCK et al., "Functional Expression of Recombinant G-Protein-Coupled Receptors Monitored by Video Imaging of Pigment Movement in Melanophores", pages 298-305. XP002921507.

## Description

### Field of the Invention

The present invention relates to an in vitro method for directly detecting a gene of a target protein to which a drug is bound in a living organism following administration of the drug to the living organism.

### Background Art

When a drug is proven to be effective for a certain disease, pharmacologists want to know, among other things, the action mechanism of the drug, i.e., which type of molecules (protein, nucleic acid, lipid, etc.) in cells the drug is bound to; how the drug alters the function of the molecules; and how the change in function relates to the efficacy of the drug. Until now, in general, intracellular target molecules, *inter alia*, target proteins of a drug have been determined through direct isolation of the molecules from the cells and tissue by use of a column to which the drug has been attached.

However, in the above method, the isolated proteins must be further purified for conversion into a single molecular species, and must be subjected to further amino acid sequence analysis. In order to determine the amino acid sequence, a protein is required typically in an amount of approximately 100 µg. Thus, a large number of cells and tissue must be employed as a starting material. Even after the amino acid sequence is determined without difficulties, isolation of the gene of the molecule and determination of the nucleotide sequence are onerous and time-consuming.

WO 96/31625A relates to the identification of an interaction of functional units with recognition units. The term recognition unit according to this document can be a peptide, carbohydrate, nucleotide and any small synthetic molecule or natural product. The antigenic substance is not further defined as being either a serum albumin or a fluorescein isothiocyanate.

WO 99/45143 relates to a method that measures fluorescence polarization of a fluorescent substance. This method does not use an antigen-antibody reaction. This document represents prior art only with regard to novelty, Art. 53 and 54 EPC.

Thus, an object of the present invention is to provide a method for directly detecting a gene of a target protein to which a drug is bound in a living organism after the drug is administered to the living organism.

### Disclosure of the Invention

In view of the foregoing, the present inventors have conducted thorough studies, and have found that a gene of a drug-targeted protein can be directly detected by causing an antigenic substance such as serum albumin to be bound to the drug via a chemical cross-linker; using the obtained material as a probe in screening the gene by use of a cDNA expression library containing a variety of genes of a drug-administered organism, e.g., a human. The present invention has been accomplished on the basis of this finding.

Accordingly, the present invention provides a method for in vitro detection of the gene of a drug-targeted protein in a living body, characterized in that the method comprises
binding an antigenic substance to the drug via a chemical crosslinker, the drug itself being a non-protein and exhibiting no antigenicity *per se*;
using the obtained material as a probe; and directly screening for a gene encoding the protein capable of binding the probe by use of a cDNA expression library containing genes of the living organism to which the drug is to be administered, wherein the screening of the gene is carried out using an antigen-antibody reaction of the antigenic substance bound to the drug and a labelled antibody directed to the antigenic substance, wherein the antigenic substance is serum albumin or fluorescein isothiocyanate.

### Best Modes for Carrying Out the Invention

The method of the present invention for detecting a gene is directed to a method for directly detecting a gene of a target protein to which a drug is bound in a living organism following administration of the drug to the living organism. The drug is administered to a living organism, preferably to mammals, particularly preferably to the human body. The drug is a non-protein substance which *per se* exhibits no antigenicity, in other words, is not immunogenic. Needless to say, a drug which exhibits no protein-binding capacity after being absorbed into the living organism cannot be employed in the present invention.

In the present invention, a substance in which an antigenic substance is bound to the drug via a chemical cross-linker is employed as a probe. No particular limitation is imposed on the chemical cross-linkers so long as they provide a group which cross-links a functional group of the drug and a functional group of the antigenic substance. Examples include glutaraldehyde, hexamethylene diisocyanate, hexamethylene diisothiocyanate, N,N'-poly(methylene)bis(iodoacetamide), N,N'-ethylenebis(maleimide), ethylene glycol bis(succinimidyl) succinate, sulfosuccinimidyl-4-(p-maleimidophenyl) butyrate, and bisdiazobenzidine. When a drug has no group which can react with such cross-linkers, an appropriate functional group must be chemically introduced into the drug. In this case, the action of the drug, such as physiological action, must not be lost. Thus, the drug is preferably a substance having a functional group which is able to react with such cross-linkers.

Preferably, the antigenic substance is a substance which *per se* has immunogenicity because the antigen-antibody reaction is advantageously employed to screen a gene which is bound to a probe. In addition, preferably, the target antibody of the antigenic substance is easily available, and the antigenic substance has a low binding property to other biocomponents. For these reasons, antigenic substances such as serum albumin and fluorescein isothiocyanate (FITC) are preferred, with bovine serum albumin (BSA) being particularly preferred. The antibody against BSA is easily available, and BSA is a predominant protein component in the blood. In addition, BSA does not readily form bonds to other biocomponents, thereby exhibiting no non-specific background during screening. Therefore, BSA is a particularly preferred antigenic substance.

The conditions of the cross-linking reaction between a drug and an antigenic substance vary in accordance with the cross-linker employed. For example, a cross-linking reaction may be carried out in a solvent at room temperature with stirring.

The cDNA expression library which is employed in the present invention preferably contains a variety of genes of mammals, particularly human genes, and examples include a human brain-originating cDNA library and a human placenta-originating cDNA library. Examples of such cDNA libraries include cDNA libraries of a vector such as plasmid or phage, with cDNA libraries containing phage as a vector being preferred and cDNA libraries containing λ phage as a vector being particularly preferred. Furthermore, cDNA libraries containing λ phage (*E. coli* as a host) as a vector are particularly preferred in view of ease of cloning. Examples of such commercially available cDNA libraries include human placenta/λTrip1 Ex library.

Screening of a target gene from the aforementioned cDNA libraries is carried out, for example, in the following manner. Specifically, a DNA library and host cells are seeded onto an agar medium, where viruses are proliferated to a certain concentration, making them produce protein. The produced protein is adsorbed and fixed on a nitrocellulose membrane. The membrane is reacted with the aforementioned probe, and plaques of probe-bound phages are detected through a chemical luminescence method employing an anti-antigenic-substance antibody (e.g., anti-BSA antibody) labeled with an enzyme such as HRP (horse radish peroxidase), serving as a secondary antibody. DNA is recovered from the thus-identified plaques, and the gene of the target protein incorporated into the DNA is analyzed using a routine method.

In another possible approach, a drug is directly labeled with an enzyme such as HRP employing a chemical cross-linker without using a secondary antibody. In this case, the enzyme activity must not be lowered due to chemical reaction.

When the gene of a drug-targeted protein is successfully analyzed, the target protein is readily identified from the deduced amino acid sequence. Examples

The present invention will next be described in detail by way of Examples, which should not be construed as limiting the invention thereto.

### Example 1

### (1) Preparation of molecular probe

As the drug, a drug (A), which is known to have excellent anti-cancer effects, represented by the following formula, was employed.

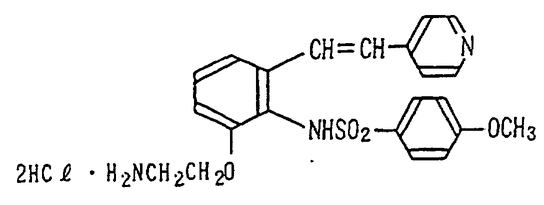

As the chemical cross linker, Sulfosuccinimidyl-4-(p-maleimidophenyl)butylate (Sulfo-SMPB) was employed. BSA was used as an antigenic substance. The compound (A) (22 mg) and Sulfo-SMPB (10 mg) were dissolved in a phosphate buffer (pH 7-9) and the resultant solution was stirred for one hour at room temperature. Subsequently, BSA (327 mg) was added thereto and the mixture was stirred at room temperature. After completion of the reaction, the mixture was desalted using a Kwik Sep™ column, to thereby yield a probe (approximately 300 mg).

### (2) Screening

A human placenta/λTrpl EX library (product of Clonetech) was employed as the cDNA library.

Firstly, in a preliminary experiment, a phage titer was determined so that about 20,000 phage plaques emerge. The phages were adsorbed onto the surface of *E. coli,* mixed with soft agar, and plated on LB agar plates (diameter: 145 mm). The number of prepared plates was 8-10. After four hours incubation at 42°C, at which point the plaques had grown to about 3-5 mm, a nitrocellulose membrane (Hybond-C Pure, diameter: 132 mm, product of Amersham) was placed carefully on the plate. Prior to placement, the membrane had been immersed in a 10 mM isopropyl-β-D-thiogalactoside (IPTG) solution for 30 minutes. In this state, the plates were incubated for an additional four hours at 37°C, to thereby produce proteins and to simultaneously cause the proteins to be adsorbed onto the membrane. Subsequently, the membranes were removed from the plates, washed with a TBST solution (the composition of which is described hereinbelow) (× 3), and subjected to blocking with a 1% gelatin solution for a period of 30 minutes to one hour. This procedure was carried out in order to suppress non-specific adsorption of antibodies onto the membranes which were to be used later. The membranes were washed with a TBST solution (× 2) and those membranes with proteins remaining attached were immersed in a TBST solution containing probes in an amount of 1/1000 at a volume ratio. In practice, the membranes and a minimum volume of the reaction mixture (1 ml or less per membrane) are put into a plastic bag together and the bag is sealed. The bag is shaken at 4°C for 12 hours or more or at room temperature for two hours or more in order to induce the proteins to react with the probes. After the reaction, the membranes are washed with a TBST solution. Subsequently, the membranes are shaken for two hours or more in a TBST solution containing a secondary antibody (HRP-labeled BSA antibody, product of Capell, anti-BSA-rabbit-antibody-peroxidase-bound IgG fraction) in an amount by volume of 1/2,500 in order to cause the proteins to react with the secondary antibody in a way similar to that when the proteins were caused to react with the probes. After washing is performed using a TBST solution, phage plaques bound to the probes were detected using a chemiluminescence ECL system (product of Amersham). The above-mentioned procedure represents the first screening. At this stage, it is impossible to recover a single plaque. Therefore, a small region of the agar medium is cut out of the plate so that each region contains plaques having emission signals (positive clones). The agar medium was immersed in an SM solution (the composition of which will be described hereinbelow) and shaken at 4°C for 12 hours or more. Subsequently, phages were recovered from the solution. The second screening was carried out using these phages. Phages having a titer that causes tens to a hundred plaques to emerge on an LB agar medium having a diameter of 85 mm were absorbed onto *E. coli,* and the phases mixed with soft agar were plated. The procedure of the second screening hereafter is the same as the first screening except for the addition of the competitor, drug A (10 µM) which was not bound to BSA, to the reaction mixture when reacting with probes. If this binding is dependant on the drug, the binding of the BSA-bound drug to the target plaque is inhibited by non-BSA-bound drugs which are added to the reaction mixture, and therefore, signals would be attenuated. The drug-specific binding was thus confirmed and a single positive clone was isolated. Hereafter, in accordance with the manual, the gene of the protein was recovered and the nucleotide sequence was determined.

HRP-labeled BSA antibody, the secondary antibody employed above was used after being subjected to absorption by Immobilized E. coli BNN97 Lysate (product of 5 Prime 3 Prime) in order to remove components of non-specific binding to virus-derived proteins. Each of the reagents used above has the following composition.
(a) TBST solution
   10 mM Tris-HCl (pH 8.0)
   150 mM NaCl
   0.05% Tween-20
(b) SM solution
   100 mM NaCl
   10 mM MgSO₄
   35 mM Tris · Cl (pH 7.5)
   0.01% Gelatin

As a result, the target proteins to which the drug (A) is bound in living organisms were found to be thymosin β-10, NF-yB, growth hormone, and glucocorticoid hormone. Among these, NF-yB is a nuclear transcription factor and thought to be difficult to isolate by the use of the customary drug column method. Therefore, where the intracellular content is low, detection can be successfully achieved using the method of the present invention.

### Industrial Applicability

The method of the present invention has eliminated the need for the protein purification step and amino acid sequence analysis, which are necessary when a conventional drug-fixed column method is performed. Also, the method of the present invention has enabled direct and simple isolation of the gene of the protein to which the drug is targeted. The invention has also enabled identification of cellular factors such as transcription factors in the nucleus, which had conventionally been difficult to purify due to the small level of such factors present in the cells.

## Claims

1. A method for in vitro detection of the gene of a drug-targeted protein in a living body, **characterized in that** the method comprises
binding an antigenic substance to the drug via a chemical crosslinker, the drug itself being a non-protein and exhibiting no antigenicity *per se;*
using the obtained material as a probe; and directly screening for a gene encoding the protein capable of binding the probe by use of a cDNA expression library containing genes of the living organism to which the drug is to be administered, wherein the screening of the gene is carried out using an antigen-antibody reaction of the antigenic substance bound to the drug and a labelled antibody directed to the antigenic substance, wherein the antigenic substance is serum albumin or fluorescein isothiocyanate.

2. A detection method according to claim 1, wherein the cDNA expression library contains a phage as a vector.

## Patentansprüche

1. Verfahren für den in-vitro-Nachweis des Gens eines Arzneimittel-Zielproteins in einem lebenden Körper, **dadurch gekennzeichnet, dass** das Verfahren folgendes umfasst:
Bindung einer Antigensubstanz an das Arzneimittel über ein chemisches Vernetzungsmittel, wobei das Arzneimittel selbst ein Nichtprotein ist und keine Antigenität per se aufweist;
Verwendung des erhaltenen Materials als Sonde und direktes Screening nach einem Gen, das das Protein codiert, das in der Lage ist, die Sonde zu binden, durch Verwendung einer cDNA-Expressionsbibliothek, die Gene des lebenden Organismus enthält, an den das Arzneimittel verabreicht werden soll, wobei das Screening des Gens unter Verwendung einer Antigen-Antikörper-Reaktion der antigenen Substanz, gebunden an das Arzneimittel, und eines markierten Antikörpers, gerichtet gegen die antigene Substanz, durchgeführt wird, wobei die antigene Substanz Serumalbumin oder Fluoreszeinisothiocyanat ist.

2. Nachweisverfahren gemäss Anspruch 1, wobei die cDNA-Expressionsbibliothek einen Phagen als Vektor enthält.

## Revendications

1. Procédé pour détection in vitro du gène d'une protéine ciblée par médicament dans un corps vivant, **caractérisé en ce que** le procédé comprend
de lier une substance antigène au médicament via un agent de réticulation chimique, le médicament lui-même étant une non protéine et ne présentant pas d'aptitude antigénique per se ;
d'utiliser le matériau obtenu en tant qu'une sonde ; et de dépister directement pour un gène codant la protéine capable de lier la sonde par l'utilisation d'une librairie d'expression d'ADNc contenant des gènes de l'organisme vivant auquel le médicament doit être administré, où le dépistage du gène est effectué en utilisant une réaction antigène-anticorps de la substance antigénique liée au médicament et un anticorps étiqueté dirigé vers la substance antigénique, où la substance antigénique est de l'albumine de sérum ou de l'isothiocyanate de fluorescéine.

2. Procédé de détection selon la revendication 1, où la librairie d'expression d'ADNc contient un phage en tant que vecteur.
